# EUROPEAN PATENT SPECIFICATION

(11) **EP 1 110 515 B1**
(45) Date of publication and mention of the grant of the patent: **22.03.2006**
(21) Application number: 01106391.4
(22) Date of filing: 26.04.1995
(51) Int. Cl.: A61F 2/06

(54) **Articulated stent**
Gelenkiger Stent
Stent articulé

(43) Date of publication of application: 27.06.2001
(62) Divisional of application: 95918298.1
(73) Proprietor: MEDINOL LIMITED, Tel Aviv 61581 (IL)
(72) Inventor: Pinchasik, Gregory, Herzelia 46424 (IL); Richter, Jacob, 47226 Ramat Hasharon (IL)
(74) Representative: Kuhnen, Rainer-Andreas

(56) References cited:
- EP-A- 0 541 443
- EP-A- 0 606 165
- US-A- 5 102 417

## Description

### FIELD AND BACKGROUND OF THE INVENTION

The present invention relates to stents which are implanted as part of a balloon angioplasty procedure within a bodily conduit of a living animal or a human to maintain patency. In particular, the present invention relates to articulated intravascular stents for delivery through or implantation in a blood vessel having a curved portion.

Intravascular stents having a constricted diameter for delivery through a blood vessel and an expanded diameter for applying a radially outwardly extending force for supporting the blood vessel are known in the art. Articulated intravascular stents for either delivery through a curved blood vessel or implanted therein are also known in the art.

Balloon-expandable stents are commercially available under the trade name Palmaz-Schatz Balloon-Expandable Stents from Johnson & Johnson Intervention Systems Co. The balloon-expandable stent described in EP-A-0 606 165 comprises a hollow tube, open at both stent ends and having a series of slots therein. The ends of the slots are rounded so as to provide smooth surfaces obviating the abrading of body passageways. However, this stent has the drawback that it shrinks significantly during expansion.

EP 0 541 443 shows a stent with two rigid end segments and a cylindrical grid of two sets of slender links crossing each other in between. The links have a helical angle of 15° in the non-expanded state and of 45° in the expanded state leading to severe foreshortening.

US A-5,102,417 to Palmaz claims to show an articulated stent with a connector between segments comprising a plurality of spiral flexible links. The links are each in the form of a straight unitary strut which is not flexible in itself.

Self-expandable articulated stents are described, for example, in U.S. Patent No. 5,104,404 entitled "Articluated Stent" to Wolff. A prior art self-expandable articulated intravascular stent **10** deployed in a curved blood vessel **16** is now described with reference to Figure 1 which is, in actual fact, Figure 2 of the above referenced U.S. Patent No. 5,104,404. Stent **10** is made up of a number of individual segments 12 articulated by hinges 14 connected a: each end to segments 12. Stent 10 is preferably fabricated from memory shape material, for example, nitinol, and as such is self expandable after delivery from a delivery system described in U.S Patent No. 4,830,003 to Wolff et al. However, these prior art articulated intravascular stents suffer from a number of disadvantages both during delivery through a curved blood vessel and when implanted therein as will now described.

The delivery of stent 10 through curved blood vessel 16 is more complicated than the delivery of a non-articulated stent in that stent 10 has to be angularly oriented such that its hinges 14 are located towards the convex portion of blood vessel 16 so that stent 10 can be flexed inward. In the present example, it will be noted that hinges 14 are located on the same side of segments 12 because blood vessel 16 has only a simple curve in one plane. It can be readily appreciated that delivery of stents through blood vessels which have one or more curved portions which are not in the same plane is even more complicated and generally requires specially constructed stents.

Even when implanted in a curved blood vessel 16, stents 10 are shown to be lacking in that the gaps between segments 12 render the curved portion of blood vessel 16 without support. Furthermore, the gaps at the convex portion of blood vessel 16 are substantially greater than the gaps at the concave portion thereof, thereby inducing non-uniform and therefore undesirable stresses on blood vessel 16.

Therefore, it would be highly desirable to have an articulated stent which does not require any particular angular orientation when being delivered through a curved bodily conduit and provides continuous and uniform support for both straight and curved portions of a bodily conduit when implanted.

It would also be highly desirable the structure of a stent does not depend on the particular orientations of curved portions of a blood vessel.

### SUMMARY OF THE INVENTION

The object of the present invention is for an articulated stent which can be delivered through a curved bodily conduit using a routine medical procedure and a conventional stent delivery system. Furthermore, the stent provides continuous and uniform support for both straight and curved portions of a bodily conduit when implanted. Still further, the structure of a stent and its support of a bodily conduit do not depend on the orientations of the curved portions of the conduit.

The objective of the present invention is achieved by an articulated stent, according to claim 1.

After expansion, the rigid segments of the stent preferably present a fine diamond shaped mesh having 1 mm long sides to provide continuous and uniform support for straight portions of a bodily conduit.

The connectors are implemented as links each having at least one kink. The connectors typically have between 8-24 links to provide continuous and uniform support for both straight and curved portions of a bodily conduit.

The stents have constricted diameters for intraluminal delivery and are then deformed, by the inflation of a balloon forming part of their catheter delivery system, to expanded diameters for applying radially outwardly extending forces for supporting the lumen of bodily conduits. The constricted and expanded diameters of the stents typically fall in the ranges of 1.0-3.5 mm and 3.5-10.0 mm, respectively.

The stents are preferably fabricated from low memory, more plastic than elastic, bio-compatible materials, for example, stainless steel 316L, gold, tantalum, etc. which enables them to be plastically deformed from their constricted diameters to their expanded diameters.

A typical stent for implantation in a human coronary artery is 9-21 mm long comprising three to seven 2.2 mm long stent segments connected by two to six 1 mm long connectors such that the ends of the stent subtend between a 45° to 135° angle at a radius of curvature of approximately 9 mm when flexed.

### BRIEF DESCRIPTION OF THE DRAWINGS

The invention is herein described, by way of example only, with reference to the accompanying drawings, wherein:

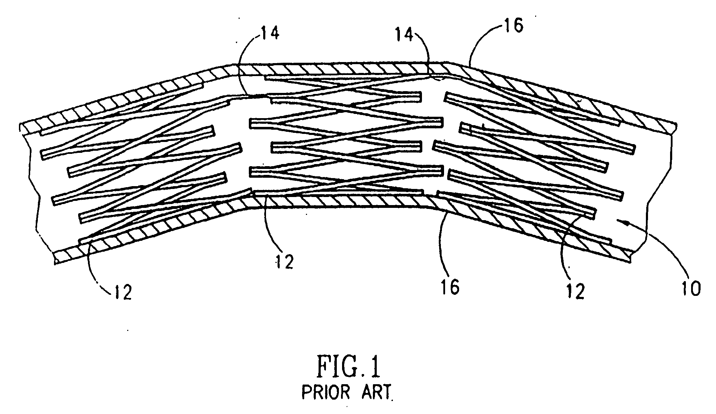

FIG. 1 shows a close-up view of a prior art articulated stent of deployed in a curved blood vessel;
FIGS. 2a and 2b show a stent not covered by the present invention but useful for illustration purposes, before plastic deformation.
FIG. 2c shows the expanded stent of Figure 2 after plastic deformation;
FIG. 2d shows the stent of Figure 2 mounted on a catheter in its flexed state;
FIGS. 2e and 2f show the stent of Figure 2 before and after expansion by a balloon forming part of its catheter delivery system;
FIGS. 3a and 3b show a preferred embodiment of an articulated stent, constructed and operative according to the teachings of the present invention, in its relaxed and flexed states before plastic deformation; and
FIG. 3c shows the expanded stent of Figure 3 after plastic deformation.

### DESCRIPTION OF THE PREFERRED EMBODIMENTS

The present invention is of an articulated stent for delivering through a curved bodily conduit, for example, a peripheral or coronary artery of a living animal or a human and implantation therein as part of a balloon angioplasty procedure to maintain patency.

The principles and operation of the articulated stent of the present invention may be better understood with reference to the drawings and the accompanying description.

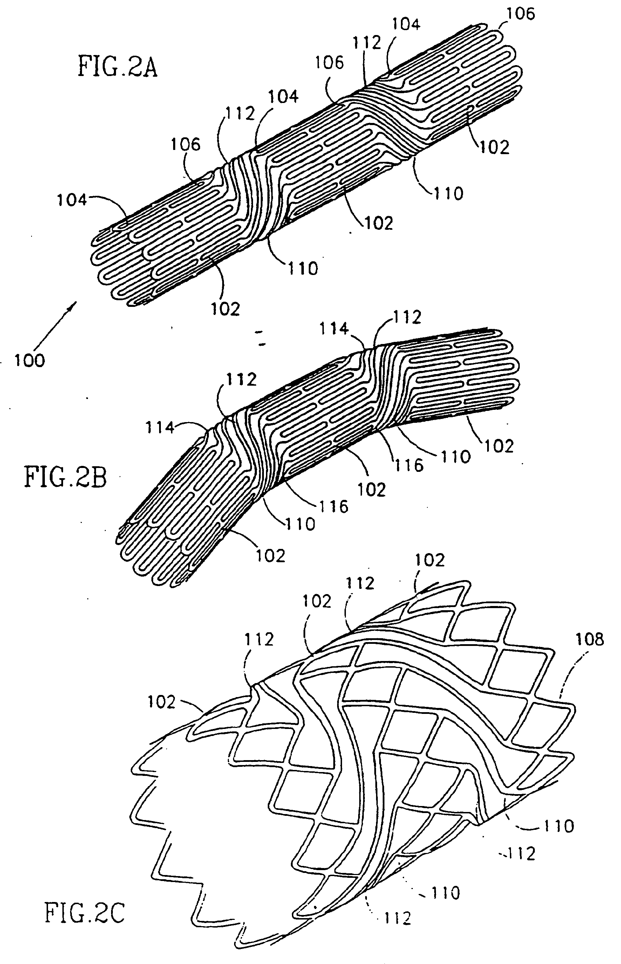

Referring now to the drawings, Figures 2a-2c show a stent not covered by the invention, generally designated **100,** generally comprising a number of substantially rigid segments **102** connected by connectors **110.**

Segments **102** are preferably made up to present a fine diamond mesh of interconnected diamond shaped cells 108 having 1 mm sides on expansion as best seen in Figure 2c. Depending on the intended diameter of stent **100,** segments **102** typically comprise between 8-24 diamond shaped cells 108.

Connectors 110 comprise links 112 connecting a front end 104 to a tail end 106 of adjacent segments 102. Links 112 preferably extend in a substantially helical fashion between apexes of diamond shaped cells **108** at front and rear ends 104 and **106** of adjacent segments **102** such that the number of links **112** equals the number of cells **108.** Links **112** are preferably evenly deployed around perimeters of segments 102 such that connectors 110 can be equally flexed in any direction and to provide continuous and uniform support to both straight and curved portions of a bodily conduit.

Alternate connectors **110** at front and rear ends **104** and **106,** respectively, of a segment **102** preferably have links **112** wound in clockwise and counter clockwise directions. Alternately winding connectors 110 ensures that the rotational displacement of links 112 and adjacent segments 102 relative to the walls of a blood vessel and more importantly the balloon of its delivery system is minimized when stent 100 is expanded.

It is particular feature of the present invention that connectors 110 have a generally cylindrical configuration when stent 100 is relaxed as best seen in Figure 2a and a differentially stretched and compressed curved configuration when stent 100 is flexed as best seen in Figure 2b. The flexed configuration is brought about by two relatively opposing displacements of links 112. First, the differential stretching of connectors 110 occurs at the convex portion thereof denoted 114 by links 112 being displaced away from one another. Second, the differential compressing of connectors **110** occurs at the concave portion thereof denoted **116** by links **112** being displaced towards one another.

Stent **100** has a constricted diameter for delivery through a curved bodily conduit as shown in Figures 2a and 2b and an expanded diameter as shown in Figure 2c for supporting a bodily conduit. Stent 100 is preferably fabricated from low memory, more plastic than elastic, bio-compatible material, for example, stainless steel 316L, gold, tantalum, etc. which enables it to be plastically deformed from its constricted diameter to its expanded diameter. The constricted and expanded diameters of stent **100** typically fall in the ranges of 1.0-3.5 mm and 3.5-10.0 mm, respectively.

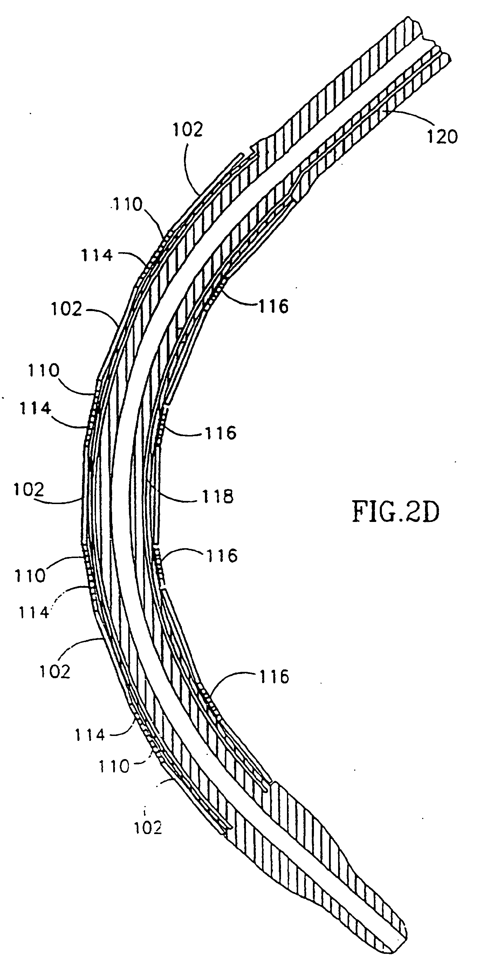

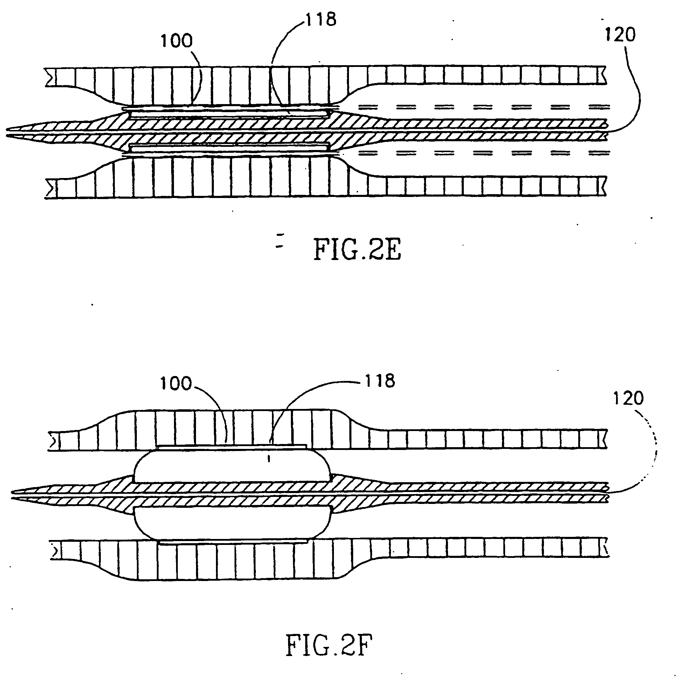

With reference now to Figures 2d-2f, stent **100** is shown overlying a balloon **118** forming part of its catheter delivery system **120.** Stent **100** is mounted on its catheter delivery system 120 in its constricted diameter state shown in Figure 2e for plastic deformation through inflation of balloon **118** to its expanded diameter shown in Figure 2f for supporting the walls of a bodily conduit. An exemplary stent for implantation in a human coronary artery, is typically 15 mm long made up of five 2.2 mm long segments **102** connected by four 1 mm long connectors 110 and capable of flexion such tha: its ends subtend a 90° angle at a radius of curvature of approximately 9 mm.

The delivery of articulated stent 100 is considerably simpler than the delivery of prior art articulated stent 10 because stent 100 is equally flexible in all direction and therefore does not require a dedicated angular orientation to pass a particular curved portion. This advantage is particularly important for delivery through blood vessels having multiple curved portions. It is a further advantage of stent 100 over prior art stents 10, that stent 100 provides continuous and uniform support along the entire length of a blood vessel by means of segments **102** and unflexed connectors **110** supporting straight portions thereof while connector portions **114** and **116** supporting convex and concave curved portions thereof, respectively.

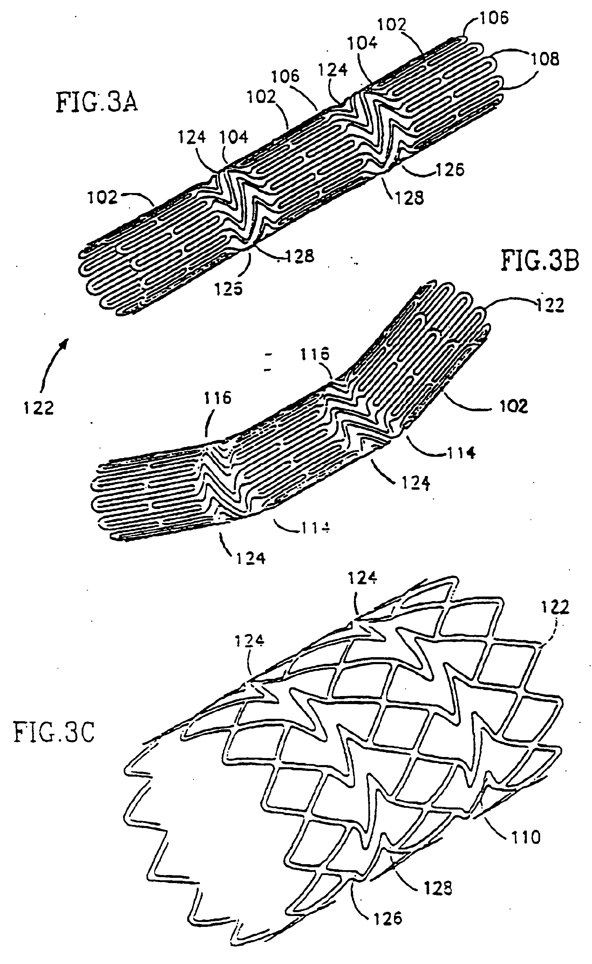

With reference now to Figures 3a and 3b, an articulated stent 122 is shown in which connectors 124 comprise links 126 having one or more kinks 128. As will readily be appreciated, the kinks 128 constitute a plurality of portions with each pair of neighbouring portions having an area of inflection there between. The design of connectors 124 is preferred to that of connector **110** because stent **100** may have a tendency to rupture balloon **118** due to two reasons. First, links **112** overlying the convex portion of balloon 118 have a tendency to be biased inward when stent **122** is flexed. Second, segments **102** display a rotational displacement relative to balloon **118** when stent **100** is expanded.

In this case, the differentially stretched and compressed curved configuration of connector 124 is brought about by two relatively opposing displacements of links 112 as before except that the differential stretching of connectors 124 at convex portion 114 occurs by kinks 128 being somewhat straightened out while the differential compressing of connectors **124** at concave portion **116** occurs by kinks **128** being more acutely bent.

In a similar fashion to stent **100,** stent **122** has a constricted diameter for delivery through a curved bodily conduit as shown in Figures 3a and 3b and an expanded diameter as shown in Figure 3c for supporting a bodily conduit when implanted therein.

While the invention has been described with respect to a limited number of embodiments, it will be appreciated that many variations, modifications and other applications of the invention may be made.

## Claims

1. A connector (124) for connecting adjacent areas of adjacent segments (102) of an articulated stent (122), the connector (124) comprising:
a plurality of flexible links (112),
**characterized in that**
each of said flexible links (112) includes a plurality of portions with each pair of neighbouring portions having a flexible area of inflection therebetween.

2. A connector (124) according to claim 1, **characterized in that** during expansion of said stent (122) said area of inflection of each flexible link (112) remains inflected.

3. An articulated stent (122) having a connector according to claim 1 or 2, and further comprising:
at least two substantially rigid segments (102) having a plurality of connected cells (108) each having apices, wherein, upon expansion, each of said rigid sements (102) presents a substantially cylindrical diamond mesh, wherein each of the flexible links (112) of said connector (124) connects apices of adjacent cells (108) on adjacent rigid segments (102).

4. The stent according to claim 3, **characterized in that** said plurality of links (112) includes between 8 and 24 links.

5. The stent according to claim 3, **characterized in that** it is made from bio-compatible material capable of a more plastic than elastic deformation.

6. The stent according to claim 5, **characterized in that** said material is stainless steel.

7. The stent according to claim 5, **characterized in that** said material is gold.

8. The stent according to claim 5, **characterized in that** said material is tantalum.

## Patentansprüche

1. Verbinder (124) zum Verbinden von benachbarten Bereichen benachbarter Segmente (102) eines Gelenk-Stents (122), wobei der Verbinder (124) aufweist:
eine Mehrzahl von flexiblen Verbindungsgliedern (112),
**dadurch gekennzeichnet, daß**
jedes der flexiblen Verbindungsglieder (112) eine Mehrzahl von Abschnitten aufweist, wobei jedes Paar von benachbarten Abschnitten einen flexiblen Biegungsbereich dazwischen aufweist,

2. Verbinder (124) nach Anspruch 1, **dadurch gekennzeichnet, daß** während der Expansion des Stents (122) der Biegungsbereich eines jeden flexiblen Verbindungsglieds (112) gebogen bleibt.

3. Gelenk-Stent (122) mit einem Verbinder nach Anspruch 1 oder 2, der ferner aufweist:
zumindest zwei im wesentlichen starre Segmente (102) mit einer Mehrzahl von verbundenen Zellen (108), die jeweils Scheitelpunkte aufweisen, wobei die starren Segmente (102) bei Expansion jeweils ein im wesentlichen zylindrisches, rautenförmiges Gitter zeigen, wobei jeder der flexiblen Verbindungsglieder (112) des Verbinders (124) jeweils Scheitelpunkte von benachbarten Zellen (108) auf benachbarten, starren Segmenten (102) verbindet.

4. Stent nach Anspruch 3, **dadurch gekennzeichnet, daß** die Mehrzahl von Verbindungsgliedern (112) zwischen 8 und 24 liegt.

5. Stent nach Anspruch 3, **dadurch gekennzeichnet, daß** er aus biokompatiblem Material gefertigt ist, das mehr zu einer plastischen als einer elastischen Verformung in der Lage ist.

6. Stent nach Anspruch 5, **dadurch gekennzeichnet, daß** es sich bei dem Material um rostfreien Stahl handelt.

7. Stent nach Anspruch 5, **dadurch gekennzeichnet, daß** es sich bei dem Material um Gold handelt.

8. Stent nach Anspruch 5, **dadurch gekennzeichnet, daß** es sich bei dem Material um Tantal handelt.

## Revendications

1. Maille de jonction (124) destinée à relier des zones adjacentes de segments adjacents (102) d'un stent articulé (122), la maille de jonction (124) comprenant :
une pluralité de maillons flexibles (112),
**caractérisée en ce que**
chacun desdits maillons flexibles (112) inclut une pluralité de parties avec chaque paire de parties voisines comportant une zone flexible entre celles-ci.

2. Maille de jonction (124) selon la revendication 1, **caractérisée en ce que** lors du déploiement dudit stent (122) ladite zone de flexion de chaque maillon flexible (112) reste fléchie.

3. Stent articulé (122) comportant une maille de jonction selon la revendication 1 ou 2, et comprenant en outre :
au moins deux segments substantiellement rigides (102) comportant une pluralité d'alvéoles reliées (108) ayant chacune des sommets, dans lequel, lors du déploiement, chacun desdits segments rigides (102) présente une maille rhombique substantiellement cylindrique, dans laquelle chacun des maillons flexibles (112) de ladite maille de jonction (124) relie des sommets d'alvéoles adjacentes (108) sur des segments rigides adjacents (102).

4. Stent selon la revendication 3, **caractérisé en ce que** ladite pluralité de maillons (112) inclut entre 8 et 24 maillons.

5. Stent selon la revendication 3, **caractérisé en ce qu'**il est fabriqué à partir d'un matériau biocompatible susceptible d'une déformation plus plastique qu'élastique.

6. Stent selon la revendication 5, **caractérisé en ce que** ledit matériau est de l'acier inoxydable.

7. Stent selon la revendication 5, **caractérisé en ce que** ledit matériau est de l'or.

8. Stent selon la revendication 5, **caractérisé en ce que** ledit matériau est du tantale.
